(19) Europäisches Patentamt
European Patent Office
Office européen des brevets

(11) EP 4 279 073 B1

(12) **EUROPEAN PATENT SPECIFICATION**

(45) Date of publication and mention of the grant of the patent:
**22.07.2026 Bulletin 2026/30**

(21) Application number: **22739028.3**

(22) Date of filing: **12.01.2022**

(51) International Patent Classification (IPC):
***A61K 31/4709*** (2006.01) ***A61K 31/045*** (2006.01)
***A61P 9/10*** (2006.01)

(52) Cooperative Patent Classification (CPC):
(C-Sets available)
**A61K 31/4709; A61K 31/045; A61P 9/10** (Cont.)

(86) International application number:
**PCT/CN2022/071541**

(87) International publication number:
**WO 2022/152150 (21.07.2022 Gazette 2022/29)**

# (54) APPLICATION OF CILOSTAZOL-CONTAINING COMPOSITION IN PREPARING DRUG FOR TREATING CEREBROVASCULAR DISEASE

VERWENDUNG EINER CILOSTAZOLHALTIGEN ZUSAMMENSETZUNG BEI DER HERSTELLUNG EINES ARZNEIMITTELS ZUR BEHANDLUNG VON ZEREBROVASKULÄREN ERKRANKUNGEN

APPLICATION D'UNE COMPOSITION CONTENANT DU CILOSTAZOL DANS LA PRÉPARATION D'UN MÉDICAMENT POUR LE TRAITEMENT D'UNE MALADIE NEUROVASCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.01.2021 CN 202110039436**

(43) Date of publication of application:
**22.11.2023 Bulletin 2023/47**

(73) Proprietor: **Neurodawn Pharmaceutical Co., Ltd.**
**Jiangsu 211199 (CN)**

(72) Inventors:
- **HUA, Yao**
  **Nanjing, Jiangsu 210046 (CN)**
- **WANG, Lei**
  **Nanjing, Jiangsu 210046 (CN)**
- **ZHANG, Zhengping**
  **Nanjing, Jiangsu 210046 (CN)**
- **CHEN, Rong**
  **Nanjing, Jiangsu 210046 (CN)**
- **YANG, Shibao**
  **Nanjing, Jiangsu 210046 (CN)**

(74) Representative: **Lavoix**
**Bayerstraße 83**
**80335 München (DE)**

(56) References cited:
**WO-A1-2017/111123 CN-A- 102 485 223**
**CN-A- 106 880 631**

- **DONALD EASTON J: "Antithrombotic Management for Transient Ischemic Attack and Ischemic Stroke (Other than Atrial Fibrillation)", CURRENT ATHEROSCLEROSIS REPORTS, CURRENT SCIENCE INC, NEW YORK, vol. 13, no. 4, 17 May 2011 (2011-05-17), pages 314 - 320, XP019921564, ISSN: 1534-6242, DOI: 10.1007/ S11883-011-0185-0**
- **MATSUMOTO ET AL: "Cilostazol in secondary prevention of stroke: Impact of the Cilostazol Stroke Prevention Study", ATHEROSCLEROSIS SUPPLEMENTS, ELSEVIER, AMSTERDAM, NL, vol. 6, no. 4, 15 December 2005 (2005-12-15), pages 33 - 40, XP027685500, ISSN: 1567-5688, [retrieved on 20051215]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **WU HAI-YIN ET AL: "The synergetic effect of edaravone and borneol in the rat model of ischemic stroke", EUROPEAN JOURNAL OF PHARMACOLOGY, ELSEVIER SCIENCE, NL, vol. 740, 5 October 2014 (2014-10-05), pages 522 - 531, XP009510121, ISSN: 0014-2999, DOI: 10.1016/J.EJPHAR.2014.06.035**
- **YAN JINLING, YAN WENHUA; LIN JINHAI: "Pharmacological effects of cilostazol and its application in ischemic stroke", CHINA PHARMACY, ZHONGHUA YIYUAN GUANLI XUEHUI, CN, vol. 23, no. 20, 23 May 2012 (2012-05-23), CN , pages 1916 - 1918, XP055950212, ISSN: 1001-0408, DOI: 10.6039/ j.issn.1001-0408.2012.20.38**
- **XU XIAOYU, ZHANG LE; YANG QIDONG: "Research progress of cilostazol in prevention and treatment of stroke", FOREIGN MEDICAL SCIENCES SECTION ON NEUROLOGY & NEUROSURGERY, vol. 40, no. 1, 28 February 2013 (2013-02-28), pages 62 - 67, XP055950220, ISSN: 1673-2642, DOI: 10.16636/ j.cnki.jinn.2013.01.019**


(52) Cooperative Patent Classification (CPC): (Cont.)

C-Sets
**A61K 31/045, A61K 2300/00;**
**A61K 31/4709, A61K 2300/00**

## Description

### CROSS REFERENCE TO RELATED APPLICATIONS

This application claims the priority of Chinese Patent Application No. 202110039436.1, filed with the China National Intellectual Property Administration on January 13, 2021, and titled with "APPLICATION OF CILOSTAZOL-CONTAINING COMPOSITION IN CEREBROVASCULAR DISEASE".

### FIELD

The present invention belongs to the field of pharmacy, and relates to use of a composition of cilostazol and (+)-2-borneol in the manufacture of a medicament for treating cerebrovascular diseases, in particular ischemic cerebrovascular diseases.

### BACKGROUND

Cerebrovascular disease (CVD) refers to brain lesions caused by various cerebrovascular diseases, and can be divided into acute cerebrovascular disease (stroke) and chronic cerebrovascular disease according to its pathogenesis process. Acute cerebrovascular diseases include transient ischemic attack, cerebral thrombosis, cerebral embolism, hypertensive encephalopathy, cerebral hemorrhage, subarachnoid hemorrhage, etc.; chronic cerebrovascular diseases include cerebral arteriosclerosis, cerebrovascular dementia, cerebral arterial steal syndrome, Parkinson's disease, etc. Ischemic stroke is a general term for brain tissue necrosis caused by insufficient cerebral blood supply due to stenosis or occlusion of cerebral blood supply arteries (carotid artery and vertebral artery). Cerebral ischemia includes four types, transient ischemic attack (TIA), reversible neurological deficit (RIND), stroke in progressive (SIE) and complete stroke (CS). There is no cerebral infarction present in TIA, but there are cerebral infarction of different degrees present in RIND, SIE and CS.

Cilostazol is an anti-platelet aggregation drug, which was first developed and synthesized by Otsuka Pharmaceutical Co., Ltd., Japan, launched in Japan in 1988, approved by FDA in the United States in May 1999 and entered China in 1996. Cilostazol is a selective inhibitor of phosphodiesterase 3 (PDE3). The binding rate of cilostazol to plasma protein is about 95%, and most of cilostazol exist in a relatively stable prototype. Cilostazol has broad-spectrum pharmacological activity and has clinical value for many diseases, such as peripheral thrombotic disease and intermittent claudication. Moreover, cilostazol has the functions of anti-platelet and vasodilation, so that it can prevent the recurrence of circulatory shock and coronary artery stenosis. Studies have shown that PDE3 can inhibit the degradation of cAMP in the circulatory system, increase cAMP in platelets and vascular smooth muscle, inhibit the formation of platelets and promote the proliferation of vascular smooth muscle cells. Cilostazol inhibits the degradation of platelets mainly by affecting the following factors: arachidonic acid, adenosine diphosphate, epinephrine, collagen and fibrinase. At present, some experts believe that cilostazol treatment can be recommended for patients with carotid artery thrombosis, which can treat or prevent cerebral ischemia. Meanwhile, PDE3 can inhibit the production of nitric oxide synthase (NOS), thereby reducing the production of nitric oxide (NO).

Cilostazol has a structure represented by the formula as follows:

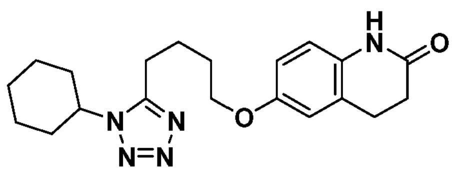

formula I

(molecular formula $C_{20}H_{27}N_5O_2$; molecular weight 369.47).

It is stipulated in the 2020 edition of the Chinese Pharmacopoeia that the main component of natural borneol, (+)-2-borneol, in natural borneol should have a content of not less than 96%. (+)-2-Borneol is a bicyclic monoterpenoid compound that exists in the volatile oils of various Chinese herbal medicines and exhibits various biological activities, such as anti-inflammation, anti-oxidation, and enhancing GABA receptor function (Euro J Pharma 2017,811:1-11). Borneol has been approved by FDA in the United States as a food flavoring agent or adjuvant (21 CFR 172.515). Borneol is also an oral adjuvant used to treat various diseases. The recommended oral dose of natural borneol for adults is 0.3-0.9 g/d (Chinese Pharmacopoeia, 2020 edition). In addition, Xingnaojing injection (which contains borneol at about 1 mg/mL according to

Drug Standards of Ministry of Health, Prepared prescription of Chinese Medicine, Volume 17) is clinically administered in a manner of intravenous infusion after diluted with 10-20 mL of 5%-10% glucose injection, or with 250-500 mL of sodium chloride injection, so that borneol is administered at an amount of 10-20 mg for a single intravenous infusion. Borneol is also an important component in Bingpeng powder, and has a content of not less than 30 mg/g (3%) (Chinese Pharmacopoeia, 2020 edition). Hai-Yin Wu et. al (The synergetic effect of edaravone and borneol in the rat model of ischemic stroke", EUROPEAN JOURNAL OF PHARMACOLOGY, ELSEVIER SCIENCE, NL, vol. 740, 5 October 2014(2014-10-05), pages 522-531) discloses the synergistic effect of edaravone and borneol in the rat model of ischemic stroke. CN 102485223A discloses the synergistic combination of (+)-2-borneol and edaravone for use in treating a cerebrovascular disease.

Therefore, it is of important practical significance to provide use of a composition comprising cilostazol in cerebrovascular diseases.

## SUMMARY

An object of the present invention is to provide use of a pharmaceutical composition in the manufacture of a medicament for treating cerebrovascular diseases, wherein the pharmaceutical composition comprises cilostazol or a pharmaceutically acceptable salt thereof and (+)-2-borneol. Further, the combined use of the pharmaceutical composition can synergistically increase the efficacy of treating cerebrovascular diseases.

The present invention provides a composition comprising the following components:

a component (I), cilostazol, a pharmaceutically acceptable salt, or a stereoisomer thereof; and

a component (II), (+)-2-borneol, or borneol;

wherein, the component (I) and the component (II) are in a weight ratio of 36:1-1:1.

In some specific embodiments of the present invention, the component (I) and the component (II) are in a weight ratio of 36:1-3:1.

In some specific embodiments of the present invention, the component (I) and the component (II) are in a weight ratio of 18:1-3:1.

In some specific embodiments of the present invention, the component (I) and the component (II) are in a weight ratio of 9:1-3:1.

In some specific embodiments of the present invention, the component (I) and the component (II) are in a weight ratio of 1:1, 3:1, 9:1, 10:1 and/or 18:1.

The present invention further provides a drug comprising the composition and a pharmaceutically acceptable adjuvant.

Based on the above research, the present invention further provides the composition or the drug for use in preventing and/or treating cerebrovascular diseases.

In some specific embodiments of the present invention, the cerebrovascular disease is selected from ischemic cerebrovascular disease; preferably, the ischemic cerebrovascular disease is selected from ischemic stroke.

The inventors of this patent found that the administration of cilostazol at 1-18 mg/kg or (+)-2-borneol at 0.27-5 mg/kg in the tail vein of rats with focal cerebral ischemia-reperfusion injury can significantly ameliorate the neurological deficits of MCAO rats and reduce the area of cerebral infarction; and the compounded combination (with a mass ratio of cilostazol: (+)-2-borneol of 18:1-1:1) within the above dosage range can produce synergistic effect. The intravenous administration of cilostazol at 10-19.46 mg/kg or (+)-2-borneol at 0.54-10 mg/kg in mice with focal cerebral ischemia-reperfusion injury can significantly ameliorate the neurological deficits and reduce the area of cerebral infarction in MCAO mice.

## DETAILED DESCRIPTION

The present invention discloses use of a composition comprising cilostazol in cerebrovascular diseases.

In the pharmaceutical composition provided by the present invention, cilostazol or a pharmaceutically acceptable salt thereof and (+)-2-borneol are in a weight ratio of 36:1-1:1; preferably, in a weight ratio of 36:1-3:1; preferably, in a weight ratio of 18:1-3:1 and 9:1-3:1; more preferably, in a weight ratio of 1:1, 3:1, 9:1, 10:1 and/or 18:1.

The pharmaceutical composition of the present invention can be used in treating cerebrovascular disease, wherein the cerebrovascular disease is preferably ischemic cerebrovascular disease, more preferably ischemic stroke.

The present invention has the following beneficial effects: according to the results of drug efficacy tests on animals (rats and mice), for cerebrovascular diseases, the combination of cilostazol and (+)-2-borneol has an effect of

synergistically increasing the drug efficacy.

The raw materials and reagents used in the use of the composition comprising cilostazol in cerebrovascular diseases were all commercially available. The present invention will be further illustrated below in conjunction with examples:

Example 1 Study on the protective effect of the composition of cilostazol and (+)-2-borneol on focal cerebral ischemia-reperfusion injury 1

1 Materials and methods

1.1 Experimental animals

Sprague-Dawley (SD) rats, male, SPF-grade, weighing 250-280 g.

1.2 Test drugs

| Sample name | Manufacturer | Batch number |
|---|---|---|
| Cilostazol | Shanghai Aladdin Biochemical Technology Co.,Ltd. | K1405071 |
| (+)-2-Borneol | Jiangsu Simovay Pharmaceutical Co., Ltd. | KC20171205-1-2 |

1.3 Experimental method

1.3.1 Animal grouping and administration

The experimental animals were divided into 4 groups, cilostazol group (1 mg/kg), (+)-2-borneol group (1 mg/kg), cilostazol and (+)-2-borneol composition group (2 mg/kg, cilostazol: (+)-2-borneol=1:1, cilostazol 1 mg/kg, (+)-2-borneol 1 mg/kg) and model group. After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

1.3.2 Establishment of focal cerebral ischemia-reperfusion model

The rat focal cerebral ischemia-reperfusion model was established by internal carotid artery suture method. The limbs (hind limbs above the knee joint and forelimbs below the wrist joint) and head of an anesthetized rat were tightened with rubber bands. The animal was fixed on an operating table in supine position, and was shaved with an animal shaver from the head to the chest, and the skin was disinfected with alcohol. The neck of the rat was cut at the midline, and the subcutaneous tissue was bluntly separated. The thin layer of fascia on the surface of the anterior triangle of the neck was separated, the lower side-lower edge of the clavicular hyoid muscle was pulled up, and the longitudinally pulsating artery parallel to this muscle can be seen. The arterial shell was opened, and the bifurcation of the right carotid artery was exposed. The right common carotid artery, external carotid artery and internal carotid artery were separated. The vagus nerve was gently stripped, and the external carotid artery was ligated and cut. The proximal end of the common carotid artery was clamped. An incision was made at the distal end from the ligature of the external carotid artery, and was inserted with a suture line, which was passed through the bifurcation of the common carotid artery into the internal carotid artery, and then was inserted slowly until reaching slight resistance (approximately 20 mm from the bifurcation), so as to block all blood supply to the middle cerebral artery. The suture line was slightly fixed below the incision of the external carotid artery with silk thread. The silk thread clamping the proximal end of the common carotid artery was loosened. A gauze soaked in sterile saline was covered on the wound, and the rat was placed on a heat preservation pad to keep warm. 2.0 h after cerebral ischemia on the right side, the suture line was pulled out gently to restore the blood supply for reperfusion. The external carotid artery was ligated with silk thread fixing the suture line. The skin was stitched, and disinfected. The rats were placed in clean feed, and their general condition and respiration were observed until they woke up from anesthesia. The rats were provided with food and water, and commonly reared.

1.3.3 Determination of the area of cerebral infarction

The animals were subjected to evaluation of neurological deficit symptoms and then sacrificed with $CO_2$. The brain was taken out by cutting off head. The olfactory bulb, cerebellum and lower brainstem were removed. The blood on the surface of the brain was washed with normal saline, and the residual water on the surface was removed. The brain was placed at -20°C for 20 min, then taken out, immediately cut to a coronal section vertically downward at the crossing plane of the line of sight, and sliced backward every 2 mm. The brain slices were incubated in 1% TTC staining solution (at 37°C for 30 min). The normal brain tissue was stained into dark red, and the ischemic brain tissue was stained into pale white. After being washed with normal saline, the brain slices were quickly arranged in a row from front to back, removed off the residual water on the surface, and photographed.

Calculation of area of cerebral infarction: The photos were processed by Image J software, and the corresponding area of left brain and non-infarction area of right brain were calculated according to the formula, so that the percentage of the area of infarction was calculated.

Calculation of volume of infarction:

$$V=t\,(A1+A2+A3+\ldots\ldots\ldots+An),$$

t is the thickness of a slice, A is the area of infarction.

$$\%I=100\%\times(VC-VL)/VC,$$

%I is the percentage of volume of infarction, VC is the brain volume of the control side (left brain), and VL is the volume of the non-infarction area of the infarction side (right brain).

1.3.4 Analysis of synergy of composition

According to Jin Zhengjun's formula $q=E(a+b)/(Ea+Eb-Ea\times Eb)$, it was evaluated whether cilostazol and (+)-2-borneol in the composition had a synergistic effect. In the formula, E(a+b) is the effective rate of combined drugs, and Ea and Eb are respectively the effective rates of drug A (cilostazol) and drug B ((+)-2-borneol) alone. $E_{administration\ group} = (X_{model}-X_{administration})/X_{model}$, wherein X is the area of cerebral infarction. q value within the range of 0.85 to 1.15 represents a simple addition of the effect of the two drugs used, q value > 1.15 represents a synergistic effect, and q value < 0.85 represents that the combined use of the two drugs has an antagonistic effect.

1.4 Statistics

Experimental data were expressed as mean ± standard deviation (Mean ± SD). Differences among groups were analyzed by one-way analysis of variance, comparison between groups was tested by LSD method, and P<0.05 was defined as significant difference.

2 Experimental results

The effects on the area of cerebral infarction are shown in Table 1. The experimental results show that the administration of (+)-2-borneol at 1 mg/kg of and the composition (cilostazol 1 mg/kg + (+)-2-borneol 1 mg/kg) can significantly reduce the area of cerebral infarction in animals (p<0.01, p<0.001), and the administration of cilostazol at 1 mg/kg tended to alleviate cerebral ischemic injury, but showed no statistical difference (p=0.06). The calculation result of synergy was q=1.24, indicating that the combined use of the two drugs had a synergistic effect.

Table 1 Effects of combined administration of cilostazol and (+)-2-borneol on the area of cerebral infarction

| Group | Number of animals | Area of cerebral infarction (%) |
|---|---|---|
| Model group | 11 | 39.73±7.62 |
| Cilostazol group | 10 | 33.78 ± 5.74 |
| (+)-2-Borneol group | 11 | 28.28 ±8.06** |
| Group of composition of cilostazol and (+)-2-borneol in 1:1 | 11 | 20.38±9.47*** |
| Mean ± standard deviation, **p<0.01, ***p<0.001, compared with the model group. | | |

Example 2 Study on the protective effect of the composition of cilostazol and (+)-2-borneol on focal cerebral ischemia-reperfusion injury 2

1 Materials and methods

1.1 Experimental animals

Sprague-Dawley (SD) rats, male, SPF-grade, weighing 250-280 g.

1.2 Test drugs

Cilostazol and (+)-2-borneol were the same as in Example 1.

1.3 Experimental method

The experimental animals were divided into 4 groups, cilostazol group (9 mg/kg), (+)-2-borneol group (1 mg/kg), cilostazol and (+)-2-borneol composition group (10 mg/kg, cilostazol: (+)-2-borneol=9:1, cilostazol 9 mg/kg + (+)-2-borneol 1 mg/kg) and model group. After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

The establishment of the focal cerebral ischemia-reperfusion model, the determination of the area of cerebral infarction, the analysis of synergy of the composition and the data statistics method were the same as in Example 1.

2 Experimental results

The effects on the area of cerebral infarction are shown in Table 2. The experimental results show that the administration of cilostazol at 9 mg/kg, (+)-2-borneol at 1 mg/kg and the composition (cilostazol 9 mg/kg + (+)-2-borneol 1 mg/kg) can significantly reduce the area of cerebral infarction in animals (p=0.024, p=0.017, p=0.000). The calculation result of synergy was q=1.5, indicating that the combined use of the two drugs had a synergistic effect.

Table 2 Effects of combined administration of cilostazol and (+)-2-borneol on the area of cerebral infarction

| Group | Number of animals | Area of cerebral infarction (%) |
|---|---|---|
| Model group | 11 | 38.25±7.87 |
| Cilostazol group | 11 | 30.00 ± 8.43* |
| (+)-2-Borneol group | 12 | 28.62±9.87* |
| Group of composition of cilostazol and (+)-2-borneol in 9:1 | 12 | 14.64±5.52*** |
| Mean ± standard deviation, **p<0.05, ***p<0.001, compared with the model group. | | |

Example 3 Study on the protective effect of the composition of cilostazol and (+)-2-borneol on focal cerebral ischemia-reperfusion injury 3

1 Materials and methods

1.1 Experimental animals

Sprague-Dawley (SD) rats, male, SPF-grade, weighing 250-280 g.

1.2 Test drugs

Cilostazol and (+)-2-borneol were the same as in Example 1.

1.3 Experimental method

The experimental animals were divided into 4 groups, cilostazol group (18 mg/kg), (+)-2-borneol group (1 mg/kg),

cilostazol and (+)-2-borneol composition group (19 mg/kg, cilostazol: (+)-2-borneol=18:1, cilostazol 18 mg/kg + (+)-2-borneol 1 mg/kg) and model group. After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

The establishment of the focal cerebral ischemia-reperfusion model, the determination of the area of cerebral infarction, the analysis of synergy of the composition and the data statistics method were the same as in Example 1.

2 Experimental results

The effects on the area of cerebral infarction are shown in Table 3. The experimental results show that the administration of cilostazol at 18 mg/kg, (+)-2-borneol at 1 mg/kg and the composition (cilostazol 18 mg/kg + (+)-2-borneol 1 mg/kg) can significantly reduce the area of cerebral infarction in animals (p=0.001, p=0.002, p=0.000). The calculation result of synergy was q=1.24, indicating that the combined use of the two drugs had a synergistic effect.

Table 3 Effects of combined administration of cilostazol and (+)-2-borneol on the area of cerebral infarction

| Group | Number of animals | Area of cerebral infarction (%) |
|---|---|---|
| Model group | 11 | 40.32±7.65 |
| Cilostazol group | 12 | 27.84 ± 7.50** |
| (+)-2-Borneol group | 11 | 29.97±7.02** |
| Group of composition of cilostazol and (+)-2-borneol in 18:1 | 12 | 15.92±7.37*** |

Mean ± standard deviation, **p<0.01, ***p<0.001, compared with the model group.

Example 4 Effects of cilostazol/(+)-2-borneol (1:1, 3:1, 9:1) on focal cerebral ischemia-reperfusion injury

1 Materials and methods

1.1 Experimental animals

Sprague-Dawley (SD) rats, male, SPF-grade, weighing 250-280 g.

1.2 Test drugs

Cilostazol and (+)-2-borneol were the same as in Example 1.

1.3 Experimental method

The experimental animals were divided into 4 groups, a model group and three groups of compositions of cilostazol/(+)-2-borneol (respectively a group of cilostazol 5 mg/kg + (+)-2-borneol 5 mg/kg in 1:1; a group of cilostazol 7.5 mg/kg + (+)-2-borneol 2.5 mg/kg in 3:1; a group of cilostazol 9 mg/kg+ (+)-2-borneol 1 mg/kg in 9:1, and each composition was administered at a total amount of 10 mg/kg). After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

The establishment of the focal cerebral ischemia-reperfusion model, the determination of the area of cerebral infarction and the data statistics method were the same as in Example 1.

2 Experimental results

The effects on the area of cerebral infarction are shown in Table 4. The experimental results show that the combined administration of cilostazol/(+)-2-borneol in 1:1, 3:1 and 9:1 can significantly reduce the area of cerebral infarction in animals (p<0.001).

Table 4 Effects of compositions of cilostazol/(+)-2-borneol on the area of cerebral infarction

| Group | Number of animals | Area of cerebral infarction (%) |
|---|---|---|
| Model group | 13 | 39.23±8.34 |
| Group of cilostazol/(+)-2-borneol in 1:1 | 11 | 22.59 ± 7.12*** |
| Group of cilostazol/(+)-2-borneol in 3:1 | 12 | 23.88±9.50*** |
| Group of cilostazol/(+)-2-borneol in 9:1 | 13 | 17.38±8.39*** |
| Mean ± standard deviation, ***p<0.001, compared with the model group. | | |

Example 5 Effects of cilostazol/(+)-2-borneol (9:1, 18:1, 36:1) on focal cerebral ischemia-reperfusion injury

1 Materials and methods

1.1 Experimental animals

Sprague-Dawley (SD) rats, male, SPF-grade, weighing 250-280 g.

1.2 Test drugs

Cilostazol and (+)-2-borneol were the same as in Example 1.

1.3 Experimental method

The experimental animals were divided into 4 groups, a model group and three groups of compositions of cilostazol/(+)-2-borneol (respectively a group of cilostazol 9 mg/kg + (+)-2-borneol 1 mg/kg in 9:1; a group of cilostazol 9.47 mg/kg + (+)-2-borneol 0.53 mg/kg in 18:1; a group of cilostazol 9.73 mg/kg + (+)-2-borneol 0.27 mg/kg in 36:1, and each composition was administered at a total amount of 10 mg/kg). After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

The establishment of the focal cerebral ischemia-reperfusion model, the determination of the area of cerebral infarction and the data statistics method were the same as in Example 1.

2 Experimental results

The effects on the area of cerebral infarction are shown in Table 5. The experimental results show that the combined administration of cilostazol/(+)-2-borneol in 9:1, 18:1 and 36:1 can significantly reduce the area of cerebral infarction in animals (p<0.001).

Table 5 Effects of compositions of cilostazol/(+)-2-borneol on the area of cerebral infarction

| Group | Number of animals | Area of cerebral infarction (%) |
|---|---|---|
| Model group | 13 | 41.08±8.52 |
| Group of cilostazol/(+)-2-borneol in 9:1 | 12 | 18.35 ± 7.86*** |
| Group of cilostazol/(+)-2-borneol in 18:1 | 12 | 23.23±10.97*** |
| Group of cilostazol/(+)-2-borneol in 36:1 | 13 | 23.40±12.11*** |
| Mean ± standard deviation, ***p<0.001, compared with the model group. | | |

Example 6 Effects of cilostazol/(+)-2-borneol (1:1, 3:1, 9:1) on focal cerebral ischemia-reperfusion injury in mice

1 Materials and methods

1.1 Experimental animals

C57BL/6J mice, male, SPF-grade, 8 week-old.

1.2 Test drugs

Cilostazol and (+)-2-borneol were the same as in Example 1.

1.3 Experimental method

The experimental animals were divided into 4 groups, a model group and three groups of compositions of cilostazol/(+)-2-borneol (respectively a group of cilostazol 10 mg/kg + (+)-2-borneol 10 mg/kg in 1:1; a group of cilostazol 15 mg/kg + (+)-2-borneol 5 mg/kg in 3:1; a group of cilostazol 18 mg/kg + (+)-2-borneol 2 mg/kg in 9:1, and each composition was administered at a total amount of 20 mg/kg). After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

Establishment of focal cerebral ischemia-reperfusion model: The mouse focal cerebral ischemia-reperfusion model was established by internal carotid artery suture method. An anesthetized mouse was fixed on an operating table in a supine position. The neck of the mice was cut at the midline, and the subcutaneous tissue was bluntly separated. The right common carotid artery, external carotid artery, and internal carotid artery were separated. The external carotid artery was inserted with a suture line, which was passed through the bifurcation of the common carotid artery into the internal carotid artery, and then was inserted slowly until reaching slight resistance (approximately 10 mm from the bifurcation), so as to block all blood supply to the cerebral artery. 60 minutes after cerebral ischemia on the right side, the suture line was gently pulled out to restore the blood supply for reperfusion. The mice were placed in clean feed, and their general condition and respiration were observed until they woke up from anesthesia. The mice were provided with food and water, and commonly reared.

The determination of the area of cerebral infarction and the data statistics method were the same as in Example 1.

2 Experimental results

The effects on the area of cerebral infarction are shown in Table 6. The experimental results show that the combined administration of cilostazol/(+)-2-borneol in 1:1, 3:1 and 9:1 can significantly reduce the area of cerebral infarction in animals (p<0.001).

Table 6 Effects of compositions of cilostazol/(+)-2-borneol on the area of cerebral infarction

| Group | Number of animals | Area of cerebral infarction (%) |
|---|---|---|
| Model group | 13 | 36.03±8.29 |
| Group of cilostazol/(+)-2-borneol in 1:1 | 11 | 21.13 ± 10.16*** |
| Group of cilostazol/(+)-2-borneol in 3:1 | 12 | 20.82±10.40*** |
| Group of cilostazol/(+)-2-borneol in 9:1 | 12 | 17.89±8.56*** |
| Mean ± standard deviation, ***p<0.001, compared with the model group. | | |

Example 7 Effects of cilostazol/(+)-2-borneol (9:1, 18:1, 36:1) on focal cerebral ischemia-reperfusion injury in mice

1 Materials and methods

1.1 Experimental animals

C57BL/6J mice, male, SPF-grade, 8 week-old.

1.2 Test drugs

Cilostazol and (+)-2-borneol were the same as in Example 1.

1.3 Experimental method

The experimental animals were divided into 4 groups, a model group and three groups of compositions of cilostazol/(+)-2-borneol (respectively a group of cilostazol 18 mg/kg + (+)-2-borneol 2 mg/kg in 9:1; a group of cilostazol 18.95 mg/kg + (+)-2-borneol 1.05 mg/kg in 18:1; a group of cilostazol 19.46 mg/kg + (+)-2-borneol 0.54 mg/kg in 36:1, and each composition was administered at a total amount of 20 mg/kg). After the cerebral ischemia model was established, the animals were assigned to each group in a single-blind manner with equal probability. After reperfusion, the animals were immediately administered intravenously with the drugs once. The animals in the model group were administered with an equal volume of normal saline. The animals were sacrificed 24 hours after cerebral ischemia, and their brains were taken out, stained, and photographed for determining the area of cerebral infarction.

The establishment of focal cerebral ischemia-reperfusion model was the same as in Example 6, and the determination of the area of cerebral infarction and the data statistics method were the same as in Example 1.

2 Experimental results

The effects on the area of cerebral infarction are shown in Table 7. The experimental results show that the combined administration of cilostazol/(+)-2-borneol in 9:1, 18:1 and 36:1 can significantly reduce the area of cerebral infarction in animals ($p<0.001$, $p<0.001$, $p<0.01$).

Table 7 Effects of compositions of cilostazol/(+)-2-borneol on the area of cerebral infarction

| Group | Number of animals | Area of cerebral infarction (%) |
|---|---|---|
| Model group | 12 | 37.99±8.59 |
| Group of cilostazol/(+)-2-borneol in 9:1 | 12 | 17.97 ± 8.70*** |
| Group of cilostazol/(+)-2-borneol in 18:1 | 13 | 22.56±10.93*** |
| Group of cilostazol/(+)-2-borneol in 36:1 | 12 | 22.49±12.96** |

Mean ± standard deviation, **p<0.01, ***p<0.001, compared with the model group.

The use of a composition comprising cilostazol in cerebrovascular diseases provided by the present invention has been described in detail above.

## Claims

1. A composition comprising the following components:

   component (I): cilostazol, a pharmaceutically acceptable salt, or a stereoisomer thereof; and
   component (II): (+)-2-borneol, or borneol;
   wherein the component (I) and the component (II) are in a weight ratio of 36:1-1:1.

2. The composition according to claim 1, wherein the component (I) and the component (II) are in a weight ratio of 36:1-3:1.

3. The composition according to claim 1, wherein the component (I) and the component (II) are in a weight ratio of 18:1-3:1.

4. The composition according to claim 1, wherein the component (I) and the component (II) are in a weight ratio of 9:1-3:1.

5. The composition according to claim 1, wherein the component (I) and the component (II) are in a weight ratio of 1:1, 3:1, 9:1, 10:1 and/or 18:1.

6. A drug, comprising the composition according to any one of claims 1 to 5, and a pharmaceutically acceptable adjuvant.

7. The composition according to any one of claims 1 to 5 or the drug according to claim 6 for use in preventing and/or treating cerebrovascular diseases.

8. The composition or the drug for use according to claim 7, wherein the cerebrovascular disease is selected from

ischemic cerebrovascular disease.

9. The composition or the drug for use according to claim 8, wherein the ischemic cerebrovascular disease is selected from ischemic stroke.

## Patentansprüche

1. Eine Zusammensetzung, die die folgenden Komponenten umfasst:

   Komponente (I): Cilostazol, ein pharmazeutisch akzeptables Salz oder ein Stereoisomer davon; und
   Komponente (II): (+)-2-borneol oder Borneol;
   wobei die Komponente (I) und die Komponente (II) in einem Gewichtsverhältnis von 36:1-1:1 vorliegen.

2. Die Zusammensetzung gemäß Anspruch 1, wobei die Komponente (I) und die Komponente (II) in einem Gewichtsverhältnis von 36:1-3:1 vorliegen.

3. Die Zusammensetzung gemäß Anspruch 1, wobei die Komponente (I) und die Komponente (II) in einem Gewichtsverhältnis von 18:1-3:1 vorliegen.

4. Die Zusammensetzung gemäß Anspruch 1, wobei die Komponente (I) und die Komponente (II) in einem Gewichtsverhältnis von 9:1-3:1 vorliegen.

5. Die Zusammensetzung gemäß Anspruch 1, wobei die Komponente (I) und die Komponente (II) in einem Gewichtsverhältnis von 1:1, 3:1, 9:1, 10:1 und/oder 18:1 vorliegen.

6. Ein Arzneimittel, das die Zusammensetzung gemäß einem der Ansprüche 1 bis 5 und ein pharmazeutisch akzeptables Adjuvans umfasst.

7. Die Zusammensetzung gemäß einem der Ansprüche 1 bis 5 oder das Arzneimittel gemäß Anspruch 6 zur Verwendung bei der Prävention und/oder Behandlung von zerebrovaskulären Erkrankungen.

8. Die Zusammensetzung oder das Arzneimittel zur Verwendung gemäß Anspruch 7, wobei die zerebrovaskuläre Erkrankung aus der Gruppe der ischämischen zerebrovaskulären Erkrankungen ausgewählt ist.

9. Die Zusammensetzung oder das Arzneimittel zur Verwendung gemäß Anspruch 8, wobei die ischämische zerebrovaskuläre Erkrankung aus der Gruppe der ischämischen Schlaganfälle ausgewählt ist.

## Revendications

1. Composition comprenant les composants suivants :

   composant (I) : cilostazol, un sel pharmaceutiquement acceptable, ou un stéréoisomère de celui-ci ; et
   composant (II) : (+)-2-bornéol, ou bornéol ;
   dans laquelle le composant (I) et le composant (II) sont dans un rapport pondéral de 36:1-1:1.

2. Composition selon la revendication 1, dans laquelle le composant (I) et le composant (II) sont dans un rapport pondéral de 36:1-3:1.

3. Composition selon la revendication 1, dans laquelle le composant (I) et le composant (II) sont dans un rapport pondéral de 18:1-3:1.

4. Composition selon la revendication 1, dans laquelle le composant (I) et le composant (II) sont dans un rapport pondéral de 9:1-3:1.

5. Composition selon la revendication 1, dans laquelle le composant (I) et le composant (II) sont dans un rapport pondéral de 1:1, 3:1, 9:1, 10:1 et/ou 18:1.

**6.** Médicament, comprenant la composition selon l'une quelconque des revendications 1 à 5, et un adjuvant pharmaceutiquement acceptable.

**7.** Composition selon l'une quelconque des revendications 1 à 5 ou médicament selon la revendication 6 pour son utilisation dans la prévention et/ou le traitement de maladies cérébrovasculaires.

**8.** Composition ou médicament pour son utilisation selon la revendication 7, dans lequel la maladie cérébrovasculaire est choisie parmi une maladie cérébrovasculaire ischémique.

**9.** Composition ou médicament pour son utilisation selon la revendication 8, dans lequel la maladie cérébrovasculaire ischémique est choisie parmi un accident vasculaire cérébral ischémique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110039436
- CN 102485223 A


### Non-patent literature cited in the description


- *Euro J Pharma*, 2017, vol. 811, 1-11
- The synergetic effect of edaravone and borneol in the rat model of ischemic stroke. **HAI-YIN WU**. EUROPEAN JOURNAL OF PHARMACOLOGY. ELSEVIER SCIENCE, 05 October 2014, vol. 740, 522-531